# EUROPEAN PATENT APPLICATION

(11) **EP 1 250 921 A1**
(43) Date of publication of application: **23.10.2002**
(21) Application number: 01109815.9
(22) Date of filing: 21.04.2001
(51) Int. Cl.: A61K 9/20, A61K 31/54

(54) **Fast disintegrating meloxicam tablet**

(71) Applicant: Boehringer Ingelheim International GmbH, 55216 Ingelheim am Rhein (DE)
(72) Inventor: Ohki, Toshimitsu, Osaka, 563-0043 (JP); Yamaguchi, Wakuko, Takarazuka, Hyogo 665-0811 (JP); Ohta, Kotoe, Kawanishi, Hyogo 666-0021 (JP)
(74) Representative: Laudien, Dieter, Dr.

(57) **Abstract**

The present invention relates to a fast disintegrating tablet comprising meloxicam or a pharmaceutically acceptable salt thereof, starch or various starches, glidant and at least one additional excipient.

## Description

### Field of the invention

The present invention relates to a fast disintegrating tablet comprising meloxicam or a pharmaceutically acceptable salt thereof, starch or various starches, glidant and at least one additional excipient.

### Background of the invention

Meloxicam [2H-1,2-benzothiazine-3-carboxamide, 4-hydroxy-2-methyl-N-(5-methyl-2-thiazolyl)-1,1-dioxide] is a non-steroidal antiinflammatory drug (NSAID) with antiinflammatory, antipyretic and analgetic activity.
Meloxicam has a low solubility in acidic or neutral medium.
The preparation of a pharmaceutical tablet formulation of meloxicam, is disclosed in PCT/EP 98/05456 as a mixing of meloxicam with special additives using different production processes ( co-milling, co-grinding, co-kneading etc.) and several processing steps to build a multilayered tablet which provides an improved solubility and bioavailability of meloxicam.
EP 0 945 134 describes the preparation of a meloxicam tablet comprising various additives and a salt of meloxicam, preferrably the meglumin salt or sodium salt of meloxicam using the direct pressing method. However the disintegration thereof in water is very slowly.

### Description of the invention

The problem underlying the present invention is to provide a tablet of meloxicam, which is able to fast disintegration in water and which is obtainable by a simple method of manufacture.

Thus it has surprisingly been found that the problem underlying the invention is solved by a tablet consisting essentially of meloxicam or a pharmaceutically acceptable salt thereof, starch or various starches, a glidant and at least one additional excipient, wherein at least one of the additional excipients is water soluble. According to the invention further auxiliary agents known in the state of the art may be used for the preparation of the tablets. Auxiliary agents are as a rule lubricants, water soluble excipients, glidants, sweeteners, souring agents and flavouring agents, which are known from the state of the art. Lubricants are preferably selected from the group consisting of magnesium stearate, stearic acid, magnesium lauryl sulfate and sodium stearyl fumarate, more preferably magnesium lauryl sulfate and magnesium stearate. Water soluble excipients are preferably selected from the group consisting of lactose, glucose, erythritol, dextrose, maltose, fructose, sucrose, sorbitol and mannitol. Glidants are preferably selected from the group consisting of hydrated silicon dioxide, talc and synthetic aluminum silicate. Sweetners are preferably selected from the group consisting of aspartame and monoammmonium glycyrrhizinate. Souring agents are preferably selected from the group consisting of mono sodium fumarate, anhydrous citric acid, citric acid, ascorbic acid and tartaric acid. Flavoring agents, which are natural or synthetic, are preferably selected from the group consisting of 1-menthol, lemon oil and orange oil.
According to the invention the term pharmaceutically acceptable salt stands for a meloxicam salt of an organic or inorganic base , as for example the meglumin, sodium, potassium or ammonium salt.

In a preferred embodiment the present invention relates to a tablet, wherein the water soluble excipients are chosen from the group comprising lactose, glucose, erythritol, dextrose, maltose, fructose, sucrose, sorbitol and mannitol, more preferably lactose or glucose, most preferably lactose.
According to the invention the term lactose stands for native lactose, lactose monohydrate or modified lactose, preferably granulated lactose monohydrate.

In a further preferred embodiment the present invention relates to a tablet, wherein glidants are chosen from the group of hydrated silicon dioxide, talc and synthetic aluminum silicate, preferably hydrated silicon dioxide.

In a further preferred embodiment of the present invention the starch or various starches are chosen from the group comprising native starch, gelatinized starch, partly gelatinized starch , starch powder, starch granules, chemically modified starch and swellable physically modified starch, preferably starch powder or partly gelatinized starch, more preferably starch powder.

Moreover the starch powder may be derived from any starch-containing plant source. This includes normal maize, hybrids like white maize, waxy maize and high-amylose-containing maizes, wheat, potato, rice, sorghum, tapioca or cassava.
In a further preferred embodiment of the present invention the starch or various starches are chosen from the group comprising rice starch, maize starch and potato starch, preferably rice starch or maize starch, most preferably maize starch or a mixture of rice starch and maize starch.

In a particularly preferred embodiment of the present invention the meloxicam salt is the sodium or meglumin salt, preferably the meglumin salt.

In a mostly preferred embodiment of the present invention the tablet consists essentially of meloxicam or a pharmaceutically acceptable salt thereof, maize starch, hydrated silicon dioxide, lactose and magnesium stearate.

In a particularly preferred embodiment of the present invention the concentration of meloxicam is 1 to 25 mg/tablet, preferably 4 to 18 mg/tablet, more preferably 5 mg/tablet, 7,5 mg/tablet, 10 mg/tablet or 15 mg/tablet, wherein the amount given relates to the active ingredient in form of the free base.

In another particularly preferred embodiment of the present invention the concentration of starch in a tablet is in the range from 20 to 50 % (w/w), preferably 25 to 48 % (w/w), more preferably 35 to 45 % (w/w), particularly preferred about 40 % (w/w).

In a further particularly preferred embodiment of the present invention the concentration of the water soluble excipient, in particular of lactose, in a tablet is in the range from 40 to 80% (w/w), preferrably 42 to 70% (w/w), more preferrably 45 to 65 % (w/w), particularly preferred about 50 % (w/w).

In a further particularly preferred embodiment of the present invention the concentration of the lubricant, in particular of magnesium stearate, in a tablet is in the range from 0,2 to 0,6% (w/w), preferrably 0,3 to 0,5% (w/w), more preferrably 0,4 % (w/w), and the concentration of glidant, in particular of hydrated silicon dioxide, in a tablet is in the range from 0,05 to 1,0 % (w/w), preferrably 0,1 to 0,5% (w/w), more preferrably 0,35 % (w/w).

More particularly preferred according to the present invention is a tablet, which is obtainable by a direct dry pressing method.

Most preferably the tablet according to the invention is free of cellulose.

As a rule the tablet according to the invention has a weight in the range of 20 to 800 mg/tablet, preferably 40 to 400 mg/tablet, more preferably 80 to 300 mg/tablet, most preferrably 90 to 260 mg/tablet, particularly preferred about 170 mg/tablet or 255 mg/tablet.

The invention will be further illustrated by the examples of tablet compositions given in Table 1. The invention should not be limited to these examples. According to the present invention the compositions are manufacturable to tablets by the direct pressing method, which is described for instance in DRUG AND THE PHARMACEUTICAL SCIENCES, Vol.71, Goeran Alderborn and Chirister Nystroem, Pharmaceutical Powder Compaction Technology, 419 - 428, Marcel Dekker, inc., Uppsala University, Sweden, 1996.
A suitable method of manufacturing the directly compressible composition is to prepare e.g. a blend of meloxicam, lactose, starch and hydrated silicon dioxide by mixing the ingredients 30 minutes in a conventional mixer. Subsequently magnesium stearate is added and the composition is blended for further 5 minutes.

**Table 1**

| Compositions of meloxicam for fast disintegrating tablets. Values are given in [mg]. | | | | | | |
|---|---|---|---|---|---|---|
| Example | 1 | 2 | 3 | 4 | 5 | 6 |
| Meloxicam | 10.0 | 10.0 | 10.0 | 10.0 | 15.0 | 15.0 |
| Lactose monohydrate | 90.7 | 90.2 | 90.7 | 91.2 | 85.7 | 136.0 |
| Maize starch | 68.0 | 68.0 | 51.0 | 34.0 | 51.0 | 76.5 |
| Rice starch | ---- | ---- | 17.0 | 34.0 | 17.0 | 25.5 |
| Hydrated silicon dioxide | 0.6 | 1.1 | 0.6 | 0.1 | 0.6 | 0.9 |
| Magnesium stearate | 0.7 | 0.7 | 0.7 | 0.7 | 0.7 | 1.1 |
| Total weight | 170.0 | 170.0 | 170.0 | 170.0 | 170.0 | 255.0 |

Disintegration experiments have been conducted according to the disintegration test, which is described in the Japanese Pharmacopoeia (JP XIII). The tested tablets were prepared by the direct pressing method from the compositions shown in Table 1.
Test results are shown in Table 2.

**Table 2.**

| Test fluid: water (the volume of the fluid in the beaker is such that, at the lowest point of the downward stroke, the top of the basket is on a level with the surface of the fluid), temperature 37+/- 2 °C. The apparatus is adjusted so as to raise and lower the basket smoothly at a constant frequency of 29 to 32 cycles per minutes. 6 tablets were tested for each batch. | | | | | | |
|---|---|---|---|---|---|---|
| Example | 1 | 2 | 3 | 4 | 5 | 6 |
| Diameter [mm] | 7 | 8 | 8 | 8 | 8 | 9 |
| Average of thickness [mm] | 3.2 | 2.7 | 2.7 | 2.7 | 2.6 | 3.1 |
| Disintegration time [sec] | 25 | 17 | 19 | 37 | 21 | 29 |

## Claims

1. A tablet, fast disintegrating in water, consisting essentially of meloxicam or a pharmaceutically acceptable salt thereof, starch or various starches, a glidant and at least one additional excipient, **characterized in that** at least one of the additional excipients is water soluble.

2. A tablet according to claim 1, **characterized in that** the water soluble excipients are chosen from the group comprising lactose, glucose, erythritol, maltose, fructose, dextrose, sucrose, sorbitol and mannitol.

3. A tablet according to claim 1 or 2, **characterized in that** the glidant is hydrated silicon dioxide.

4. A tablet according to one of claims 1 to 3, **characterized in that** the starch or various starches are chosen from the group comprising native starch, gelatinized starch, partly gelatinized starch , starch powder, starch granules, chemically modified starch and swellable physically modified starch.

5. A tablet according to one of claims 1 to 4, **characterized in that** the starch or various starches are chosen from the group comprising rice starch, maize starch and potato starch.

6. A tablet according to one of claims 1 to 5, **characterized in that** the starch is maize starch or a mixture of maize and rice starch.

7. A tablet according to one of claims 1 to 5, consisting essentially of meloxicam or a pharmaceutically acceptable salt thereof, maize starch or a mixture of maize and rice starch, hydrated silicon dioxide, lactose and magnesium stearate.

8. A tablet according to one of claims 1 to 7, comprising 1 to 20 mg of meloxicam in form of the free base.

9. A tablet according to one of claims 1 to 8, **characterized in that** the concentration of starch in a tablet is in the range from 20 to 50 % (w/w).

10. A tablet according to one of claims 1 to 9, **characterized in that** the concentration range of the water soluble excipient in a tablet is in the range from 40 to 80% (w/w).

11. A tablet according to one of claims 1 to 10, **characterized in that** the tablet is produced by a direct dry pressing method.

12. A tablet according to one of claims 1 to 11, **characterized in that** it is free of cellulose.

13. A tablet according to one of claims 1 to 12, **characterized in that** the weight of the whole tablet is in the range of 20 to 800 mg.
